# EUROPEAN PATENT SPECIFICATION

(11) **EP 2 911 780 B1**
(45) Date of publication and mention of the grant of the patent: **10.03.2021**
(21) Application number: 13795183.6
(22) Date of filing: 25.10.2013
(51) Int. Cl.: B01J 13/22, C11D 3/50

(54) **CAPSULES**
KAPSELN
CAPSULES

(30) Priority: 25.10.2012 EP 12290370
(43) Date of publication of application: 02.09.2015
(73) Proprietor: Givaudan SA, 1214 Vernier (CH); Sorbonne Université, 75006 Paris 6 (FR); Centre National de la Recherche Scientifique, 75794 Paris Cedex 16 (FR)
(72) Inventor: BONE, Stephane, 69580 Sathonary Village (FR); GEFFROY, Cèdric, 96180 Buxerolles (FR); LE TIRILLY, Sandrine, 75013 Paris (FR); PERRIN, Patrick, 75014 Paris (FR); VAUTRIN, Claire, 38580 Pinsot (FR); MONTEUX, Cécile, 75231 Paris (FR); PANTOUSTIER, Nadège, 75231 Paris (FR)
(74) Representative: Global Patents
(86) International application number: PCT/EP2013/072403
(87) International publication number: WO 2014/064255

(56) References cited:
- EP-A1- 1 393 706
- EP-A1- 1 589 092
- EP-A1- 1 721 963
- EP-A2- 1 719 554
- EP-A2- 1 797 947
- WO-A1-2004/016234
- WO-A2-2009/138978
- US-A1- 2007 138 671
- DATABASE WPI Week 200978 Thomson Scientific, London, GB; AN 2009-Q28525 XP002723252, & JP 2009 241044 A (FUJI FILM CO LTD) 22 October 2009 (2009-10-22)

## Description

The present invention is concerned with encapsulation of beneficial agents being fragrances.

Core-shell capsules are known in the art. They generally consist of a polymeric shell that is formed around a core containing a beneficial agent, such as a fragrance, and indeed any other ingredients that should be encapsulated.

Core-shell capsules are typically formed by forming droplets of core-forming material dispersed in an aqueous continuous phase. Monomers that should react to form a polymeric shell around the core droplets are contained in the droplets and/or the continuous phase. They can be made to react to form polymeric coatings around the droplets, which are subsequently consolidated (hardened) by crosslinking reactions. In this way, it is possible to form core-shell capsules comprising shells formed of aminoplast and resins, in particular thermoset resins, polyureas, polyurethanes, polyamides, gelatin, polyacrylics and the like.

US 2007/138671 A1 discloses a microcapsule comprising a core comprising an active material and a shell formed of an acrylamide-acrylic acid co-polymer. The active material is a perfume. The acrylamide-acrylic acid co-polymer has a molecular weight of from 5 000 to 1 000 000.

Core-shell capsules are very versatile. The polymeric shell may have a barrier function, protecting the core contents from the environment external of the capsule, but it may also act as a means of modulating the release of fragrance at a desired rate of release. The nature and composition of the shell can influence capsule properties such as hardness, brittleness and porosity, and as such, affect the manner in which the beneficial agent can be released therefrom. A disadvantage of forming shells by in-situ polymerisation reactions is that the formed capsules can contain significant levels of unreacted monomers. Residues of monomers, for example, can be particularly undesirable in contact with skin, for instance in Personal Care applications. For this reason it is conventional to carry out numerous post-treatments on capsules to remove these residues after high conversion polymerisation, which is very difficult to achieve during interfacial polymerization. Post-treatment techniques may take the form of post-reacting with another molecule or post curing, volatilization, stream stripping, molecular trapping or extraction using a suitable solvent or ion-exchange resins. Unfortunately, these techniques are not particularly suitable when the capsules contain volatile benefit agents such as fragrances.

There remains a need to provide core-shell capsules, wherein the shell is based on polymers, which capsules contain very low levels of monomer residues.

Applicant has found that it is possible to form shells of pre-formed polymeric material based on polycarboxylic acids, around oil droplets comprising a fragrance. As the polymeric material is laid down pre-formed, capsules can be formed that contain extremely low levels of monomeric material heretofore unachievable using conventional in-situ polymerisation techniques.

The invention provides in a first aspect core-shell capsules comprising a fragrance-containing core and a shell formed around said core, wherein the shell is formed of at least one layer of polycarboxylic acid having a molecular weight of 1'000 to 10'000'000 Daltons, and wherein the shell consists of multiple layers of polycarboxylic acid, and wherein each layer of polycarboxylic acid is interposed with a layer of a different polymeric material, the polymer combinations of the shell being poly(acrylic acid) of 50,000g/mol/poly(vinyl pyrrolidone) of 40,000 g/mol, or poly(methacrylic acid)/ poly(vinyl pyrrolidone).

The capsules of the present invention are distinct from conventional polyacrylic capsules in that the shell is formed of pre-formed polymeric material as opposed to the conventional approach whereby monomers are reacted in situ to form the shell. In the latter case, the shell consists of a polymeric mass with no defined or measurable molecular weight.

There are several advantages attendant to the present invention. By employing pre-formed polymers to form the shell, one can exert great control over the shell's mechanical properties by selecting the desired molecular weight of the component polymers. Furthermore, the use of pre-formed polymeric materials substantially avoids the problem of residual monomeric contaminants being found in the capsules.

Accordingly, in another aspect of core-shell capsules comprising a fragrance-containing core and a shell formed around said core, wherein the shell is formed of polycarboxylic acid and an interposed different polymer as hereinabove described, and wherein any carboxylic acid monomer is present in an amount of 100 ppm or less, more particularly 10 ppm or less, still more particularly 1 ppm or less.

Residual carboxylic acid can be measured by techniques known in the art, for example by GC analysis. Typically, a GC column may contain polydimethylsiloxane and the carrier gas may be helium. Monomeric residues can be extracted with a suitable solvent such as methanol or the like.

The polymeric material forming said interposed layer or layers is able to interact with the adjacent polycarboxylic acid layer by forming hydrogen bonds.

By selecting the desired numbers of layers it is possible to closely control the thickness of the capsule shell. In a particular embodiment of the present invention the shell is from 5 to 150 nm thickness. By selecting the thickness of the shell as well as the nature of the pre-formed polymer material forming it, one is able to tailor the properties of the capsule shell in terms of porosity, robustness (to withstand the rigours of manufacture, compounding, transportation and storage) as well as friability (to permit the capsule to rupture in use at the desired time to release its fragrance).

The core-shell capsules are somewhat sensitive to high pH conditions. Indeed, at pH levels at 6.5 or above, the shells will begin to dissociate back into the polymeric material from which they were formed.

Accordingly, the disclosure provides in another of its aspects a method of stabilising said core-shell particles by storing them in a suitable medium at a pH of less than about 5, more particularly at a pH of about 2 to 4.

In another aspect of the invention there is provided an aqueous slurry comprising core-shell capsules as herein described, wherein said slurry has a pH of less than about 5, more particularly 2 to 4.

The slurry may contain 5 to 50 % by weight of core-shell capsules, more particularly 10 to 40%, still more particularly 20 to 30 % by weight. Optionally, the slurry may contain a stabilizing polymer. It is common to employ stabilising polymers in slurries containing capsules and the skilled person would understand the common types of stabilising polymers that can be employed. A particular stabilising polymer is Keltrol. The stabilising polymer can be employed in amounts of about 0.05 to 5 % by weight, more particularly 0.1 to 0.3%. The remaining mass of the slurry typically is comprised of water.

Having regard to the pH sensitivity of the core-shell capsules of the present invention, it is preferred if they are employed in applications in which the pH of the surrounding medium is 2 to 7. Core shell capsules of the present invention are particularly suitable for use in laundry applications including softeners, personal care and hair care applications including shampoo, conditioners, combing creams, leave on conditioners, styling cream, soaps, body creams; deodorants and anti-perspirants; household applications, fine fragrance and oral care.

The technique of forming a shell around a core by the sequential deposition of layers of polymer around said core is known in the art. Previously, however, this technique has involved the alternate application of two polyelectrolytes to a sacrificial template core (typically silica) to build up a series of layers held together by electrostatic attraction. When the desired number of layers is attained, the core is then dissolved to give a hollow capsule, which may then be loaded with the desired beneficial agent. The technique offers the possibility of unprecedented control over release properties, and has been of great interest to the pharmaceutical industry (see, for example, Kozlovskaya et al, Chem. Mater. 2006, 18, 328-336).

In accordance with the present invention, it has been found possible to make core-shell capsules around liquid droplets containing a beneficial agent without the need of any kind of template or sacrificial core. Forming capsule shells around liquid droplets is more challenging than forming shells around solid cores because the former is a more mobile phase and ingredients can partition between the droplets and the external phase during the process of shell formation. Fragrances are particularly challenging to encapsulate in this way because their overall character depends on them consisting of complex, multi-component mixtures, which components have different physical properties and partition at different rates and to different extents.

Core-shell capsules formed according to the present invention are useful for encapsulating, among other useful beneficial agents, fragrance ingredients. The capsules can be formed with minimum loss of core material and maintenance of the hedonic character of the fragrance core created by the perfumer.

In forming a core-shell capsule by causing layers of polymeric material to be laid down on droplets of dispersed phase, once the initial two layers have been applied around said droplets, each succeeding layer need not be selected from the same materials as the initial two layers. The only requirement is that each succeeding layer is capable of interacting either physically or chemically with the layer adjacent it. This allows considerable versatility in how a capsule may be prepared in order to achieve a particular desired result.

The polymeric materials may be applied to droplets of a dispersed phase by introducing polymeric material to be deposited into the external phase surrounding the droplets under conditions permitting deposition of the polymeric material on the droplets. For example, the polymeric material can be introduced into the external phase in an amount of about 0.2 to 10 % w/w in the external phase, more particularly 1 to 5 % by weight, and at a pH of about 2 to 6. The amount of polymeric material introduced will depend upon its molecular weight (the higher the molecular weight, generally the lower amounts that will be employed in the external phase).

Different layers of polymeric material can be laid down sequentially, if after deposition of a layer of polymeric material is complete, the excess of that polymeric material is washed out of the external phase with water and a new (successive) polymeric material is introduced into the external phase. This process is repeated until the desired number of layers has been deposited. The process has the advantage that no particular proportions of polymer need be used, so no precise compositional limitations need be observed. The core-shell capsules may be formed in this manner using techniques including spray-coating or microfluidics.

Once the shell is formed, optionally, it may be crosslinked. This can be achieved by any convenient means. For example, polymers having amino or hydroxyl groups may be crosslinked by the addition of materials such as polyaldehyde, polyfunctional isocyanates or epoxides or any other suitable crosslinker.

Should it be desired, the shell thus formed can be used as a templating agent around which chemistry may be carried out to form aminoplast, polyurea, polyurethane, polyacrylic, inorganic microcapsules or hybrid capsules.

The disclosure is further described with reference to the following non-limiting examples.

### Example 1:

### Preparation of fragrance-containing capsules

5mL of a 1% (wt) poly (methacrylic acid) [PMAA] of 100'000 g/mol molecular weight aqueous solution is adjusted to pH to 3.. To this solution is added 5mL of a proprietary fragrance. The two solutions are kept in contact for 24 hours, and then emulsified using an Ultra-turrax™ blender at 24000 rpm for 2 minutes. The emulsion is added to a separating funnel and washed with water at pH 3 (10⁻³ M HCl) to give a 10% fragrance solution.

This solution is slowly mixed and then allowed to settle for 24 hours.
The lower aqueous phase is removed and washed four times with water at pH=3 to extract excess polymer. This washed phase is added to i(wt)% aqueous PVP (poly (vinyl pyrrolidone) of 40,000g/mol molecular weight) solution at pH3 under gently stirring. This phase is allowed to settle for 24h and it is then washed with water at pH 3 as described in the previous step. The settling, lower layer removal and washing steps are repeated.

The previous step is repeated with PMAA solution, and then again with PVP solution. This alternation is continued until 5 layers have been deposited.

The final fragrance-containing capsules have an average diameter of 31µm and a wall thickness of about 14nm.

### Example 2:

### Preparation of fragrance-containing capsules

The polymers used in this example are poly(acrylic acid) [PAA]of 50,000 g/mol molecular weight and poly(vinyl pyrrolidone) of 40,000g/mol molecular weight.

5mL of a 1 % (wt) PAA aqueous solution is adjusted to pH to 3. To this solution is added 5mL of a proprietary fragrance. The two solutions are kept in contact for 24 hours, and then emulsified using an Ultra-turrax™ blender at 24000rpm for 2 minutes. The emulsion is added to a separating funnel and washed with water at pH 3 (10⁻³ M HCl) to give a 10 % fragrance solution. This solution is slowly mixed and then allowed to settle for 24 hours.

The lower aqueous phase is removed and washed four times with water at pH=3 to extract excess polymer. This washed phase is added to i(wt)% aqueous PVP (poly (vinyl pyrrolidone) of 40,000g/mol molecular weight) solution at pH3 under gently stirring. This phase is allowed to settle for 24h and it is then washed with water at pH 3 as described in the previous step. The settling, lower layer removal and washing steps are repeated.

The previous step is repeated with PAA solution, and then again with PVP solution. This alternation is continued until 5 layers are attained.

The final fragrance-containing capsules have an average diameter of 31µm and a wall thickness of about 18nm

### Example 3:

### Polymer recovery

1g of capsule dispersion from Example 1 and Example 2 are introduced separately in 100g of water at pH= 8 to 9 under slow stirring. A white dispersion thus formed, first turns translucent and then transparent whilst an oily phase appears on the surface of the vessel when stirring is stopped.

The water phase is analysed by size exclusion chromatography using PL Aquagel-OH 8µm (300x7.5mm) and water at pH=7 with 0.2M NaNO3 + 0.01 M NaH2PO4 as eluent, PEG as calibration curve. Pure polymers used to manufacture capsule were analysed as well:

| Samples | Mw of Peak 1 (g/mol) | Mw of Peak 2 (g/mol) |
|---|---|---|
| Pure PMAA | 109,000 | - |
| Pure PVP | 45,000 | - |
| Pure PAA | 55,000 | - |
| Example 1 after recovery | 44,000 | 108,000 |
| Example 2 after recovery | 45,000 | 55,000 (overlapping) |

This demonstrates that polymers used in the formation of microcapsules are recovered after destruction of the capsules at a pH of between 8 and 9. This establishes that the capsule shell consists of discrete polymeric units of a defined molecular weight.

### Example 4:

Preparation of a flavour-shifting ice cream comprising 10-layer PMAA/PVP microcapsules of Example 1.

An ice cream is prepared by mixing the microcapsules containing a cherry flavour into an ice cream formulation at pH 5 containing a vanilla flavour.

The initial taste sensation is vanilla, quickly followed by cherry flavour after dissolution of capsules at higher pH in the mouth.

## Claims

1. Core-shell capsules comprising a fragrance-containing core and a shell formed around said core, wherein the shell is formed of at least one layer of polycarboxylic acid having a molecular weight of 1'000 to 10'000'000 Daltons, and wherein the shell consists of multiple layers of polycarboxylic acid, and wherein each layer of polycarboxylic acid is interposed with a layer of a different polymeric material, the polymer combinations of the shell being poly(acrylic acid) of 50,000g/mol/poly(vinyl pyrrolidone) of 40,000 g/mol, or poly(methacrylic acid)/ poly(vinyl pyrrolidone).

2. Core-shell capsules according to claim 1 wherein any carboxylic acid monomer contained in the capsules is present in an amount of 100 ppm or less, more particularly 10 ppm or less, still more particularly 1 ppm or less.

3. Core-shell capsules according to any of the preceding claims wherein said polycarboxylic acid is poly(methacrylic acid) and said interposed polymeric material is poly(vinyl pyrrolidone).

4. An aqueous slurry comprising core-shell capsules as defined in any of the preceding claims wherein said slurry has a pH of less than 6, more particularly from 2 to 6, more particularly from 2 to 4.

5. An aqueous slurry according to claim 4 comprising core-shell capsules, a stabiliser and a buffer.

6. A consumer product composition having a pH of less than 6, more particularly from 2 to about 6, more particularly from 2 to 4, selected from laundry cleaners and softeners, personal care and hair care products including shampoo, conditioners, combing creams, leave on conditioners, styling cream, soaps, body creams; deodorants and anti-perspirants; household applications, fine fragrance and oral care products, comprising core-shell capsules or aqueous slurry containing core-shell capsules as defined in any of the claims 1 to 3.

## Patentansprüche

1. Kern-Schale-Kapseln, umfassend einen duftstoffhaltigen Kern und eine um den Kern herum gebildete Schale, wobei die Schale aus mindestens einer Schicht aus Polycarbonsäure mit einem Molekulargewicht von 1000 bis 10.000.000 Dalton gebildet ist und wobei die Schale aus mehreren Schichten von Polycarbonsäure besteht und wobei zwischen jeder Schicht aus Polycarbonsäure eine Schicht aus einem anderen Polymermaterial eingefügt ist, wobei es sich bei den Polymerkombinationen der Schale um Poly(acrylsäure) mit einem Molekulargewicht von 50.000 g/mol/Poly(vinylpyrrolidon) mit einem Molekulargewicht von 40.000 g/mol oder Poly(methacrylsäure)/Poly(vinylpyrrolidon) handelt.

2. Kern-Schale-Kapseln nach Anspruch 1, wobei jegliches in den Kapseln enthaltene Carbonsäure-Monomer in einer Menge von 100 ppm oder weniger, spezieller 10 ppm oder weniger, noch spezieller 1 ppm oder weniger, vorliegt.

3. Kern-Schale-Kapseln nach einem der vorhergehenden Ansprüche, wobei es sich bei der Polycarbonsäure um Poly(methacrylsäure) handelt und es sich bei dem eingefügten Polymermaterial um Poly(vinylpyrrolidon) handelt.

4. Wässrige Aufschlämmung, umfassend Kern-Schale-Kapseln gemäß einem der vorhergehenden Ansprüche, wobei die Aufschlämmung einen pH-Wert von weniger als 6, spezieller von 2 bis 6, spezieller von 2 bis 4, aufweist.

5. Wässrige Aufschlämmung nach Anspruch 4, umfassend Kern-Schale-Kapseln, einen Stabilisator und einen Puffer.

6. Konsumproduktzusammensetzung mit einem pH-Wert von weniger als 6, spezieller von 2 bis 6, spezieller von 2 bis 4, ausgewählt aus Wäschereinigern und -weichspülern, Körperpflege- und Haarpflegeprodukten einschließlich Shampoo, Konditionierungsmitteln, Kämmcremes, Leave-on-Konditionierungsmitteln, Styling-Creme, Seifen, Körpercremes; Deodorantien und Antiperspirantien; Haushaltsanwendungen, Feinduftstoffen und Mundpflegeprodukten, umfassend Kern-Schale-Kapseln oder wässrige Aufschlämmung enthaltend Kern-Schale-Kapseln gemäß einem der Ansprüche 1 bis 3.

## Revendications

1. Capsules de type noyau-enveloppe comprenant un noyau contenant une fragrance et une enveloppe formée autour dudit noyau, l'enveloppe étant formée d'au moins une couche d'un poly(acide carboxylique) possédant un poids moléculaire de 1 000 à 10 000 000 Daltons, et l'enveloppe étant constituée de plusieurs couches de poly(acide carboxylique), et chaque couche de poly(acide carboxylique) étant intercalée avec une couche d'un matériau polymérique différent, les combinaisons de polymère de l'enveloppe étant poly(acide acrylique) de 50 000 g/mole/polyvinylpyrrolidone de 40 000 g/mole, ou poly(acide méthacrylique)/polyvinylpyrrolidone.

2. Capsules de type noyau-enveloppe selon la revendication 1, tout monomère de type acide carboxylique contenu dans les capsules étant présent en une quantité de 100 ppm ou moins, plus particulièrement 10 ppm ou moins, encore plus particulièrement 1 ppm ou moins.

3. Capsules de type noyau-enveloppe selon l'une quelconque des revendications précédentes, ledit poly(acide carboxylique) étant un poly(acide méthacrylique) et ledit matériau polymérique intercalé étant une polyvinylpyrrolidone.

4. Suspension aqueuse comprenant des capsules de type noyau-enveloppe telles que définies dans l'une quelconque des revendications précédentes, ladite suspension possédant un pH inférieur à 6, plus particulièrement de 2 à 6, plus particulièrement de 2 à 4.

5. Suspension aqueuse selon la revendication 4 comprenant des capsules de type noyau-enveloppe, un stabilisant et un tampon.

6. Composition de produit de consommation possédant un pH inférieur à 6, plus particulièrement de 2 à environ 6, plus particulièrement de 2 à 4, choisie parmi des produits de nettoyage et des assouplissants de blanchisserie, des produits de soin personnel et de soin capillaire y compris un shampooing, des après-shampooings, des crèmes capillaires, des après-shampooings sans rinçage, une crème coiffante, des savons, des crèmes corporelles ; des déodorants et des antitranspirants ; des applications ménagères, des produits de parfumerie fine et de soin buccal, comprenant des capsules de type noyau-enveloppe ou une suspension aqueuse contenant des capsules de type noyau-enveloppe telles que définies dans l'une quelconque des revendications 1 à 3.
